(19) Europäisches Patentamt
European Patent Office
Office européen des brevets

(11) Veröffentlichungsnummer **0 003 041**
**B1**

(12) # EUROPÄISCHE PATENTSCHRIFT

(45) Veröffentlichungstag der Patentschrift
**10.06.81**

(51) Int Cl³ **A 61 K 43/00**

(21) Anmeldenummer. **78101838.7**

(22) Anmeldetag **23.12.78**

(54) **Technetium-99m-markiertes Diagnostikum zur Darstellung des retikuloendothelialen Systems (RES) und Verfahren zu seiner Herstellung.**

(30) Priorität **07.01.78 DE 2800538**

(43) Veröffentlichungstag der Anmeldung
**25.07.79 Patentblatt 79/15**

(45) Bekanntmachung des Hinweises auf die Patenterteilung
**10.06.81 Patentblatt 81/23**

(84) Benannte Vertragsstaaten
**BE CH DE FR GB IT LU NL SE**

(56) Entgegenhaltungen
**Keine**

(73) Patentinhaber **HOECHST AKTIENGESELLSCHAFT,**
**Zentrale Patentabteilung Postfach 80 03 20,**
**D-6230 Frankfurt/Main 80 (DE)**

(72) Erfinder **Strecker, Helmut, Dr.,**
**Wilhelm-Leuschner-Strasse 43,**
**D-6104 Seeheim-Jugenheim (DE)**
Erfinder **Molter, Michael, Dr., Unter den Akazien 7,**
**D-6000 Frankfurt/Main 70 (DE)**
Erfinder **Kloss, Gerhard, Dr., Liederbachstrasse 14,**
**D-6233 Kelkheim (Taunus) (DE)**
Erfinder **Schickel, Eberhard, Am Weidenbruch 24,**
**D-6251 Selters (DE)**

**EP 0 003 041 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Technetium-99m-markiertes Diagnostikum zur Darstellung des retikuloendothelialen Systems (RES) und Verfahren zu seiner Herstellung

Technetium-99m wird aufgrund seiner günstigen Strahlenenergie von 140 keV, seiner relativ kurzen Halbwertszeit von 6 Stunden und der Abwesenheit von Korpuskularstrahlung in der Nuklearmedizin für diagnostische Zwecke eingesetzt. In der Regel wird es, um einen zu großen Verlust an Radioaktivität — durch Zerfall während des Transports — zu vermeiden, aus einem sogenannten Nuklidgenerator (wie z. B. in DOS 2 236 565 beschrieben) direkt in der Klinik gewonnen.

Das aus einem solchen Nuklidgenerator erhaltene Technetium-99m (es liegt als $NaTcO_4$ in 0,9% NaCl-Lösung vor) kann nur bedingt direkt zur medizinischen Diagnostik eingesetzt werden, und zwar hauptsächlich zur Hirn-, Schilddrüsen- und Magenszintigraphie. In der Regel müssen für weitere Untersuchungen geeignete organspezifische Transportsubstanzen für das Technetium-99m mit diesem Radionuklid »markiert« werden. Als Transportsubstanzen dienen z. B. Partikel je nach Größe für die Lungen- und Leberdiagnostik und Pyrophosphat für die Skelettdarstellung.

Die Markierung dieser Transportsubstanzen erfolgt in der Regel nach Reduktion des als reaktionsträges Pertechnetat vorliegenden Technetium-99m zu sehr reaktionsfreudigen niedrigeren Oxidationsstufen (wahrscheinlich 4 oder 5). Die Reduktion kann im sauren (z. B. mit Thiosulfat), im Neutralen (im Wesentlichen mit Zinn (II)) und auf elektrolytischem Wege erfolgen. Im ersten Fall muß die Reaktionslösung vor der Injektion noch neutralisiert werden. Das führt zu einem sog. »Mehrkomponentenbesteck« (s. u.). Die elektrolytische Reduktion ist apparativ aufwendig.

Die Technetium-99-m-Markierung erfolgt in der Klinik, um Zerfallsverluste an Radioaktivität zu vermeiden. Deshalb ist ein einfaches, schnelles und sicheres Verfahren angebracht. Da das Präparat in der Regel injiziert wird, muß es steril, pyrogenfrei und nicht toxisch sein. Zunehmend setzen sich Markierungsbestecke durch. Das sind aufeinander abgestimmte Geräte und inaktive Substanzen, die in Kombination mit dem Nuklidgeneratorprodukt zu einem organspezifischen Diagnostikum führen. Besonders leicht zu handhaben ist als Markierungsbesteck eine sogenannte »Markierungseinheit« (Einkomponentenbesteck): ein Injektionsfläschen mit einer Substanzkombination, in das das Generatorprodukt nur noch injiziert werden muß, um das gebrauchsfertige Diagnostikum zu erhalten.

Solche Markierungseinheiten bestehen häufig aus einer Kombination einer organspezifischen Trägersubstanz mit einem Zinn(II)-salz.

Substanzen und Bestecke zur Diagnostik des RES (Leber, Milz und Knochenmark) sind bekannt, z. B.

1. Gold-198-Kolloid
2. Technetium-99m-Schwefel-Kolloid
3. Technetium-99m-Zinn-Hydroxid
4. Technetium-99m-Zinn-Phytat
5. Indium-113m-Eisenhydroxid-Partikel

Alle diese Verfahren haben Nachteile, die im folgenden dargestellt werden:

Gold-198-Kolloid zeichnet sich zwar durch seine homogene Teilchengröße positiv aus; Gold-198 führt aber aufgrund seiner im Vergleich zum Technetium-99m ungünstigen Strahleneigenschaften (2,7 Tage Halbwertszeit, Korpuskularstrahlung) zu einer höheren Strahlenbelastung beim zu untersuchenden Patienten.

Technetium-99m-Schwefel-Kolloid ist bezüglich seiner diagnostischen Eignung ebenfalls ein Präparat mit guter Qualität; zu seiner Präparation ist aber ein relativ kompliziertes Verfahren notwendig: Das Eluat muß zunächst angesäuert, mit Thiosulfat versetzt, dann gekocht und schließlich neutralisiert werden. Damit ist eine erhöhte Strahlenbelastung des Personals verbunden.

Ein Kolloid, das nur aus Zinnhydroxid-Partikeln besteht, kann zwar hergestellt werden, bei ihm besteht aber die Gefahr, daß die Partikel aggregieren (Alterungsprozesse). Es läßt sich dann nach Zugabe der Pertechnetatlösung kein brauchbares Kolloid mehr gewinnen; außerdem reichern sich die relativ großen Partikelaggregate in der Lunge an.

Technetium-99-Phytat kann ebenfalls auf einfache Weise aus einer Markierungseinheit hergestellt werden. Es ist aber nicht genau bekannt, nach welchem Prinzip das Präparat in der Leber angereichert wird. Vermutlich reagiert das Phytat bei der iv.-Injektion mit den Kalziumionen des Blutes unter Partikelbildung. Die Qualität der Partikel hängt somit von variablen Größen, wie Kalziumgehalt des Blutes und Injektionsgeschwindigkeit ab, so daß das physiologische und somit auch das diagnostische Verhalten des Präparates schwankt.

Indium-113m-Eisenhydroxid-Partikel haben bezüglich ihres physiologischen Verhaltens und der hohen Strahlenenergie des Indium-113m, die schlechter für Untersuchungen mit der $\gamma$-Kamera geeignet ist, Nachteile.

Aufgabe der vorliegenden Erfindung ist die Herstellung eines Leberdiagnostikums das die Nachteile der oben genannten Diagnostika vermeidet.

Gegenstand der Erfindung ist ein Verfahren zur Herstellung eines Diagnostikums zur Darstellung des retikuloendothelialen Systems (RES), insbesondere der Leber, das dadurch gekennzeichnet ist, daß man 1-Phenyl-2,3-dimethyl-pyrazolon-4-methylamino-methylsulfonsaures Natrium (A) oder 1-Phenyl-2,3-dimethyl-5-pyrazolon (B)

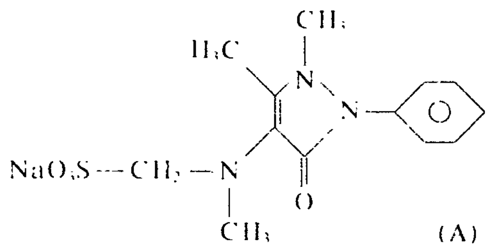

(A)

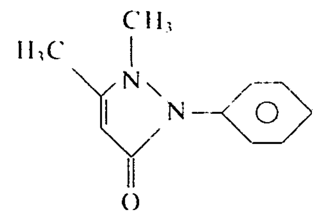

(B)

in wäßriger Lösung mit Zinn(II)-Salz in einem Molverhältnis zwischen 15 : 1 und 110 : 1 mischt, die Lösung auf einen pH-Wert zwischen 4 und 9, vorzugsweise zwischen 5,5 und 6,5 einstellt, gegebenenfalls die Mischung lyophilisiert, und anschließend je nach Verwendungszweck 0,1 bis 100 mCi Technetium-99m-Pertechnetat in 1—10 ml physiologischer Kochsalzlösung zugibt. Die Konzentrationen von (A) und (B) liegen zweckmäßig zwischen 0,1 und 200 mg/ml, vorzugsweise zwischen 1 und 50 mg/ml.

Für die Herstellung des Diagnostikums ist es von Vorteil, wenn (A) bzw. (B) bei einem pH-Wert unter 2, vorzugsweise bei 1 bis 1,5 mit dem Zinn(II)-Salz in Lösung gemischt wird und anschließend mit Alkalilauge langsam unter intensivem Rühren auf einen pH-Wert zwischen 4 und 9, vorzugsweise zwischen 5,5 und 6,5, eingestellt wird. Etwa ab pH 3,5 trübt sich die bis dahin klare Lösung durch die Bildung der Zinn-(A)- bzw. Zinn-(B)-Partikeln ein.

Daß es sich bei dem vorliegenden Diagnostikum nicht lediglich um eine Mischung von Zinn(II)-Salz mit (A) bzw. (B) handelt, geht eindeutig aus dem Verhalten der einzelnen Komponenten und der Gemische bei Änderung des pH-Wertes hervor. Wäßrige Lösungen von (A) und (B) sind im gesamten pH-Bereich zwischen 2 und 11,5 klar. Bei Lösungen von Zinn(II)-Salz mit und ohne (A) bzw. (B), die bei niedrigen pH-Werten alle klar sind, setzt ab pH 3,5 eine Trübung ein, die ihr Maximum bei ca. pH 4,5 erreicht. Lösungen, die nur Zinn(II)-Salz enthalten, werden oberhalb solcher Lösungen, die Zinn(II)-Salz und (A) oder Zinn(II)-Salz und (B) enthalten, bis zu pH-Werten über 11 trübe bleiben.

Die Partikelsuspensionen werden entweder innerhalb von 24 Stunden verwendet, oder sie werden zur Stabilisierung bei Temperaturen unter 0°C, vorzugsweise zwischen −5 und −20°C und einem Druck unter 2 Torr, vorzugsweise zwischen 0,2 und 0,01 Torr, gefriergetrocknet und anschließend unter einem inerten Schutzgas, z. B. Stickstoff, gehalten.

Gegenstand der Erfindung ist weiterhin ein Diagnostikum zur Darstellung des retikuloendothelialen Systems, insbesondere der Leber, das dadurch gekennzeichnet ist, daß es kolloidale (A)- oder (B)-Zinn-Technetium-99m-Verbindungen in physiologischer Kochsalzlösung enthält.

## Beispiel 1

1 g (A) wird in ca. 80 ml bidest. Wasser gelöst. Es werden dann 20 mg $SnCl_2 \cdot 2\,H_2O$ in 1 ml 0,1 N HCl gelöst und zu der Lösung von (A) zugegeben. Nach dem Durchmischen wird unter Rühren mittels NaOH der pH-Wert auf 6,0 eingestellt und mit bidest. Wasser auf das Endvolumen von 100 ml aufgefüllt. Der pH-Wert wird gegebenenfalls noch einmal korrigiert, die Endlösung wird dann in 1 ml-Portionen vereinzelt und zur Markierung mit 0,1 bis 100 mCi, vorzugsweise 2—20 mCi $^{99m}TcO_4^-$, in 1—10 ml physiolog. Kochsalzlösung versetzt. Das Diagnostikum soll innerhalb eines Arbeitstages injiziert werden.

Alle verwendeten Lösungen werden mit Stickstoff sauerstofffrei geblasen sein. Das Ansetzen der Lösung und das Vereinzeln geschieht unter Sauerstoff-Ausschluß. Zur Herstellung der Lösungen zur Anwendung bei Menschen werden sterile und pyrogenfreie Substanzen und Lösungsmittel verwendet.

(A) = 1-Phenyl-2,3-dimethyl-pyrazolon-4-methylamino-methylsulfonsaures Natrium.

## Beispiel 2

5 g (B) werden in ca. 900 ml bidest. Wasser gelöst und mit 200 mg $SnCl_2 \cdot 2\,H_2O$ in 2 ml 0,1 N HCl versetzt. Nach dem Durchmischen wird unter kräftigem Rühren mit Alkalilauge (z. B. NaOH) der pH-Wert auf 6,0 eingestellt und mit bidest. Wasser auf das Endvolumen von 1000 ml aufgefüllt. Der Ansatz wird dann 2 Stunden unter Sauerstoffausschluß gerührt und die Suspension in 1 ml-Portionen in mit flüssigem Stickstoff vorgefrorene Injektionsflaschen vereinzelt.

Die auf diese Weise eingefrorenen Portionen werden ohne zwischenzeitliches Auftauen bei einer

Plattentemperatur von $-10°C$ und einem Druck von $<0,1$ Torr gefriergetrocknet. Nach dem vollständigen Trocknen werden die Injektionsflaschen mit Stickstoff gefüllt und verschlossen. Man erhält so eine zur Markierung mit Technetium-99m geeignete Packungseinheit (Markierungseinheit).

Zu einer Markierungseinheit werden bei Bedarf je nach Anwendungszweck 0,1 bis 100 mCi, vorzugsweise 2—20 mCi, $^{99m}TcO_4$- in 1—10 ml physiologischer Kochsalzlösung zugegeben. Das so zubereitete fertige Diagnostikum soll innerhalb von 24 Stunden appliziert werden.

Alle verwendeten Lösungen werden mit Stickstoff sauerstofffrei geblasen. Das Ansetzen der Lösung und das Vereinzeln geschehen unter Sauerstoff-Ausschluß. Die Lösungen sind zur Anwendung beim Menschen steril und pyrogenfrei, da sie aus sterilen Substanzen und Lösungsmittel hergestellt werden.

(B) = 1-Phenyl-2,3-dimethyl-pyrazolon

## Patentansprüche

1. Verfahren zur Herstellung eines Diagnostikums zur Darstellung des retikuloendothelialen Systems, insbesondere der Leber, das dadurch gekennzeichnet ist, daß man 1-Phenyl-2,3-dimethyl-pyrazolon-4-methylamino-methylsulfonsaures Natrium (A) oder 1-Phenyl-2,3-dimethyl-5-pyrazolon (B) in wäßriger Lösung mit Zinn(II)-Salz in einem Molverhältnis zwischen 15 : 1 und 110 : 1 mischt, die Lösung auf einen pH-Wert zwischen 4 und 9, vorzugsweise zwischen 5,5 und 6,5 einstellt, gegebenenfalls die Mischung lyophilisiert, und anschließend je nach Verwendungszweck 0,1 bis 100 mCi Technetium-99m-Pertechnetat in 1—10 ml physiologischer Kochsalzlösung zugibt.

2. Verfahren nach Anspruch 1, dadurch gekennzeichnet, daß die Konzentrationen von (A) und (B) zweckmäßig zwischen 0,1 und 200 mg/ml, vorzugsweise 1 und 50 mg/ml liegt.

3. Verfahren nach Anspruch 2, dadurch gekennzeichnet, daß eine Markierungseinheit 1—50, vorzugsweise 5 bis 10 mg (A) oder (B), sowie 0,02 bis 1, vorzugsweise 0,1 bis 0,2 mg $SnCl_2 \cdot 2 H_2O$ enthält, und mit 1—10 ml physiologischer Technetium-99m-Pertechnetatlösung zur Reaktion gebracht wird.

4. Verfahren nach Ansprüchen 1 und 2, dadurch gekennzeichnet, daß (A)- oder (B)-Zinn-Technetium-99m-Verbindungen in physiologischer Kochsalzlösung verwendet werden.

5. Diagnostikum zur Darstellung des retikuloendothelialen Systems, insbesondere der Leber, das dadurch gekennzeichnet ist, daß es eine kolloidale Lösung von 1-Phenyl-2,3-dimethyl-pyrazolon-4-methylamino-methylsulfonsaures Natrium (A) oder 1-Phenyl-2,3-dimethyl-5-pyrazolon (B) als Zinn-Technetium-99m-Verbindung in physiologischer Kochsalzlösung enthält.

## Claims

1. A process for the preparation of a diagnostic agent for scanning the RES, especially that of the liver, which comprises mixing the sodium salt of 1-phenyl-2,3-dimethyl-pyrazolone-4-methylamino-methylsulfonic acid (A) or 1-phenyl-2,3-dimethyl-5-pyrazolone (B) in an aqueous solution with a tin(II) salt in a molar ration of 15 : 1 to 110 : 1, adjusting the resulting solution to a pH between 4 and 9, preferably between 5.5 and 6.5, optionally lyophilizing it and afterwards adding from 0.1 to 100 mCi of $^{99m}$Tc-pertechnetate in 1 to 10 ml of physiological saline solution.

2. A process as claimed in claim 1, wherein the concentration of (A) or (B) is in the range of from 0.1 to 200 mg/ml, preferably from 1.0 to 50 mg/ml.

3. A process as claimed in claim 1 or 2, which comprises reacting a labelling unit containing from 1 to 50 mg, preferably from 5 to 10 mg of (A) or (B) and from 0.02 to 1 mg preferably from 0.1 to 0.2 mg of $SnCl_2 \cdot 2 H_2O$ with $^{99m}$Tc-pertechnetate in 1 to 10 ml of physiological saline solution.

4. A process as claimed in claim 1 or 2, wherein (A) or (B)-tin-$^{99m}$Tc compounds are contained in a physiological saline solution.

5. Diagnostic agent for visualizing the RES, especially that of the liver, containing a colloidal (A) or (B)-tin $^{99m}$Tc compound in physiological saline solution.

## Revendications

1. Procédé de préparation d'un agent de diagnostic pour la visualisation du système réticuloendothélial, en particulier du foie, caractérisé en ce qu'on mélange le sel de sodium de l'acide 1-phényl-2,3-diméthyl-pyrazolon-4-méthylamino-méthylsulfonique (A) ou la 1-phényl-2,3-diméthyl-5-pyrazolone (B) en solution aqueuse avec un sel d'étain (II) en un rapport molaire compris entre 15 : 1 et 110 : 1, on ajuste la solution à une valeur de pH compris entre 4 et 9, de préférence entre 5,5 et 6,5, on lyophilise éventuellement et enfin selon l'application on ajoute de 0,1 à 100 mCi de pertechnétate marqué au technétium-99m dans 1 à 10 ml d'une solution physiologique de NaCl.

2. Procédé selon la revendication 1, caractérisé en ce que les concentrations de (A) et de (B) sont avantageusement comprises entre 0,1 et 200 mg/ml et de préférence entre 1 et 50 mg/ml.

3. Procédé selon la revendication 2, caractérisé en ce qu'une unité de marquage contient de 1 à 50, de préférence de 5 à 10 mg de (A) ou de (B), ainsi que de 0,02 à 1, de préférence de 0,1 à 0,2 mg de $SnCl_2 \cdot 2\,H_2O$ et est amenée à réagir avec de 1 à 10 ml d'une solution physiologique de pertechnétate marqué au technétium-99m.

4. Procédé selon les revendications 1 et 2, caractérisé en ce qu'on utilise des combinaisons de (A)-ou de (B)-étain-technétium 99m dans une solution physiologique de NaCl.

5. Agent de diagnostic pour la visualisation du système réticuloendothélial, en particulier du foie, caractérisé en ce qu'il contient une solution colloidale du sel de sodium de l'acide 1-phényl-2,3-diméthyl-pyrazolon-4-méthylamino-méthyl sulfonique (A) ou de 1-phényl-2,3-diméthyl-5-pyrazolone (B) sous forme de combinaison étain-technétium-99m dans une solution physiologique de NaCl.